# EUROPEAN PATENT APPLICATION

(11) **EP 4 137 510 A1**
(43) Date of publication of application: **22.02.2023**
(21) Application number: 21789230.6
(22) Date of filing: 16.04.2021
(51) Int. Cl.: C07K 14/705, C07K 19/00, C12N 15/09, A61K 38/17, A61P 35/00, A61P 37/00

(54) **PD-1 MUTANT POLYPEPTIDE, AND PREPARATION THEREFOR AND USE THEREOF**

(30) Priority: 16.04.2020 CN 202010299304
(71) Applicant: Nantong Yichen Biopharma. Co. Ltd., Nantong, Jiangsu 226001 (CN)
(72) Inventor: WANG, Feng, Shanghai 200233 (CN); ZHENG, Huayang, Shanghai 200233 (CN); ZHANG, Yuhan, Shanghai 200233 (CN)
(74) Representative: V.O.
(86) International application number: PCT/CN2021/087864
(87) International publication number: WO 2021/209049

(57) **Abstract**

The present application provides a high-affinity PD-1 mutant polypeptide, and a fusion protein of the PD-1 mutant polypeptide and an antibody (specifically binding to a tumor antigen) and preparation and application thereof.

## Description

### Technical Field

The present invention relates to the field of biopharmaceuticals, and in particular to a high-affinity PD-1 mutant polypeptide, and an antibody fusion protein containing the PD-1 mutant polypeptide and preparation and application thereof.

### Background

PD-1 is an immunosuppressive molecule that belongs to the CD28 family. Its structure includes: an extracellular immunoglobulin variable region (IgV)-like domain, a hydrophobic transmembrane region, and an intracellular region. PD-1 is expressed on CD4-CD8-thymocytes, and inducibly expressed on activated T cells, B cells, bone marrow cells, dendritic cells, natural killer cells, monocytes etc. The continuous expression of PD-1 on T cells may induce T cell exhaustion. The expression of PD-1 by tumor-infiltrating lymphocytes may affect T cell function, weaken cytokine secretion, and weaken the tumor-killing effect of T cells, and is closely related to the poor prognosis and high tumor recurrence rate of patients with renal cell carcinoma and non-small cell lung cancer (Pan Jiajia, et al.; Journal of China Pharmaceutical University 2016, 47(1):9-18).

PD-1 has two ligands, namely PD-L1 and PD-L2, both of which belong to the B7 family. PD-L1 is widely expressed in activated B cells, T cells, macrophages, dendritic cells (DCs), NK cells etc. PD-L1 is also expressed on the surface of many tumor cells, such as lung cancer, breast cancer, malignant melanoma, esophageal cancer, gastric cancer, and pancreatic cancer. The high expression of PD-L1 or PD-L2 on surface of tumor cells binds to PD-1 on T cells to transduce negative regulatory signals, leading to the immune apoptosis and immune incompetence of tumor antigen-specificity T cells, and making tumor cells escape from the immune surveillance and killing (Pan Jiajia et al.; Journal of China Pharmaceutical University 2016, 47(1):9-18). Therefore, with PD-1/PD-Ls signaling pathway as the target, the development of blockers against PD-1 or PD-Ls can enhance the killing of tumor cells by T cells.

At present, there are three immune checkpoint inhibitors targeting PD-L1 on the market, but the epitopes of these known PD-L1 antibodies are not completely consistent with the key domains of PD-L1 binding to PD-1.Although these inhibitors can block the interaction of PD-1 and PD-L1, their effects on tumor cells are different from that of PD-1 and its derivatives that specifically bind to PD-L1. In addition, the protein folding, structure, post-translational modification and the resulting protein physicochemical properties and in vivo metabolic processes of the PD-1 extracellular domain are different from those of antibody domain. These differences will make drugs containing the PD-1 extracellular domain have functional characteristics that the antibodies cannot replace. Therefore, high-affinity PD-1 analogues that can specifically interact with PD-L1 has great clinical application prospects in anti-tumor immunity.

### Summary

Compared with the affinity of wild-type PD-1 and PD-L1 (nM), the PD-1 mutant polypeptide (with 2 amino acid mutations) of the present invention not only retains the physicochemical properties and the same epitope binding similar to that of wild-type PD-1, but also greatly improves its affinity to PD-L1 (pM), and the possibility of off-target is low; in addition, the PD-1 mutant polypeptide can cross-react with PD-L1 from human, monkey and mouse, providing great convenience for studies on pharmacodynamics and toxicology in animal models.

Compared with PD-L1 antibodies, the PD-1 mutant polypeptide has a single domain, and is easy to construct bispecific antibodies. The antibody fusion protein formed by fusing the PD-1 mutant polypeptide with the antibody targeted to a tumor antigen, on the one hand, can specifically confine the PD-1 mutant polypeptide on or near tumor cells and in the tumor microenvironment through high-affinity ((higher than the affinity of the PD-1 mutant polypeptide with PD-L1)tumor antigen-specificity antibody, greatly reducing the extensive immune activation caused by conventional immune checkpoint inhibitors in vivo; and on the other hand, the fast binding and fast dissociation characteristics of the PD-1 mutant polypeptide enable it to block the PD-1/PD-L1 signaling pathway, avoiding the adverse reactions resulting from continuous activation of immune cells by the fast binding and slow dissociation properties of the conventional immune checkpoint inhibitors. In addition, the faster in vivo metabolism of the PD-1 mutant than that of conventional antibody fragments helps to enhance its activities on tumors and shorten the retention time in normal tissue and plasma, which is helpful for improving Therapeutic Index (TI).

In an aspect of the present invention, a PD-1 mutant polypeptide is provided. In some embodiments, the PD-1 mutant polypeptide has a nucleic acid sequence as shown in SEQ ID NO: 1, or has an amino acid sequence as shown in SEQ ID NO: 2.

In an aspect of the present invention, an antibody fusion protein containing the PD-1 mutant polypeptide is provided. In some embodiments, the antibody fusion protein includes the PD-1 mutant polypeptide and an antibody targeted to a tumor cell surface antigen. In some embodiments, the tumor cell surface antigen is selected from EGFR, HER2, HER3, GPC3, PSMA, CD27, CD33, CD37, CD38, CD51, CD56, mesothelin, HGFR, Nectin-4, Mucin 5AC, folate receptor α, carbonic anhydrase IX (CAIX), DLL1, Notch2, Notch3 and endoglin etc.

In some embodiments, the tumor antigen is selected from EGFR. In some embodiments, the antibody targeted to the tumor cell surface antigen EGFR has sequences HCDR1, HCDR2 and HCDR3 as shown in SEQ ID NO: 13, SEQ ID NO: 14, and SEQ ID NO: 15, and sequences LCDR1, LCDR2 and LCDR3 as shown in SEQ ID NO: 16, SEQ ID NO: 17, and SEQ ID NO: 18. In some embodiments, the antibody targeted to the tumor antigen EGFR has a heavy chain variable region (VH) with an amino acid sequence as shown in SEQ ID NO: 34, and a light chain variable region (VL) with an amino acid sequence as shown in SEQ ID NO: 36. In some embodiments, the antibody targeted to the tumor cell surface antigen EGFR has a VH with an amino acid sequence as shown in SEQ ID NO: 65, and a VL with an amino acid sequence as shown in SEQ ID NO: 66. In some embodiments, the antibody targeted to the tumor antigen EGFR has a heavy chain (HC) with an amino acid sequence as shown in SEQ ID NO: 4, and a light chain (LC) with an amino acid sequence as shown in SEQ ID NO: 6 . In some embodiments, the antibody fusion protein containing the PD-1 mutant polypeptide and the antibody targeted to the tumor cell surface antigen EGFR has a HC with an amino acid sequence as shown in SEQ ID NO: 4 and a LC with an amino acid sequence as shown in SEQ ID NO: 28.

In some embodiments, the tumor antigen is selected from HER2. In some embodiments, the antibody targeted to the tumor cell surface antigen HER2 has sequences HCDR1, HCDR2 and HCDR3 as shown in SEQ ID NO: 19, SEQ ID NO: 20, and SEQ ID NO: 21, and sequences LCDR1, LCDR2 and LCDR3 as shown in SEQ ID NO: 22, SEQ ID NO: 23, and SEQ ID NO: 24. In some embodiments, the antibody targeted to the tumor cell surface antigen HER2 has a VH with an amino acid sequence as shown in SEQ ID NO: 38, and a VL with an amino acid sequence as shown in SEQ ID NO: 40. In some embodiments, the antibody targeted to the tumor cell surface antigen HER2 has a VH with an amino acid sequence as shown in SEQ ID NO: 67, and a VL with an amino acid sequence as shown in SEQ ID NO: 68. In some embodiments, the antibody targeted to the tumor antigen HER2 has an HC with an amino acid sequence as shown in SEQ ID NO: 8 and an LC with an amino acid sequence as shown in SEQ ID NO: 8. In some embodiments, the antibody fusion protein containing the PD-1 mutant polypeptide and the antibody targeted to the tumor antigen HER2 has a HC with an amino acid sequence as shown in SEQ ID NO: 10 and a LC with an amino acid sequence as shown in SEQ ID NO: 32.

In some embodiments, the tumor cell surface antigen is selected from GPC-3. In some embodiments, the antibody targeted to the tumor cell surface antigen GPC3 has sequences HCDR1, HCDR2, and HCDR3 as shown in SEQ ID NO: 45, SEQ ID NO: 46, and SEQ ID NO: 47, and sequences LCDR1, LCDR2, and LCDR3 as shown in SEQ ID NO: 48, SEQ ID NO: 49, and SEQ ID NO: 50. In some embodiments, the antibody targeted to the tumor cell surface antigen GPC-3 has a VH with an amino acid sequence as shown in SEQ ID NO: 42, and a VL with an amino acid sequence as shown in SEQ ID NO: 44. In some embodiments, the antibody targeted to the tumor antigen GPC-3 has an HC with an amino acid sequence as shown in SEQ ID NO: 58 and an LC with an amino acid sequence as shown in SEQ ID NO: 60. In some embodiments, the antibody fusion protein containing the PD-1 mutant polypeptide and the antibody targeted to the tumor antigen GPC-3 has a HC with an amino acid sequence as shown in SEQ ID NO: 52 and a LC with an amino acid sequence as shown in SEQ ID NO: 60. In some embodiments, the antibody fusion protein containing the PD-1 mutant polypeptide and the antibody targeted to the tumor antigen GPC-3 has a HC with an amino acid sequence as shown in SEQ ID NO: 58 and a LC with an amino acid sequence as shown in SEQ ID NO: 54. In some embodiments, the antibody fusion protein containing the PD-1 mutant polypeptide and the antibody targeted to the tumor antigen GPC-3 has a HC with an amino acid sequence as shown in SEQ ID NO: 58 and a LC with an amino acid sequence as shown in SEQ ID NO: 56.

In some embodiments, the tumor cell surface antigen is selected from PSMA. In some embodiments, the antibody targeted to the tumor cell surface antigen PSMA has a VH with an amino acid sequence as shown in SEQ ID NO: 62, and a VL with an amino acid sequence as shown in SEQ ID NO: 64.

In some embodiments, the tumor cell surface antigen is selected from CD27. In some embodiments, the antibody targeted to the tumor cell surface antigen CD27 has a VH with an amino acid sequence as shown in SEQ ID NO: 69, and a VL with an amino acid sequence as shown in SEQ ID NO: 70.

In some embodiments, the tumor cell surface antigen is selected from CD33. In some embodiments, the antibody targeted to the tumor cell surface antigen CD33 has a VH with an amino acid sequence as shown in SEQ ID NO: 71, and a VL with an amino acid sequence as shown in SEQ ID NO: 72.

In some embodiments, the tumor cell surface antigen is selected from CD37. In some embodiments, the antibody targeted to the tumor cell surface antigen CD37 has a VH with an amino acid sequence as shown in SEQ ID NO: 73, and a VL with an amino acid sequence as shown in SEQ ID NO: 74.

In some embodiments, the tumor cell surface antigen is selected from CD38. In some embodiments, the antibody targeted to the tumor cell surface antigen CD38 has a VH with an amino acid sequence as shown in SEQ ID NO: 75, and a VL with an amino acid sequence as shown in SEQ ID NO: 76.

In some embodiments, the tumor cell surface antigen is selected from CD51. In some embodiments, the antibody targeted to the tumor cell surface antigen CD51 has a VH with an amino acid sequence as shown in SEQ ID NO: 77, and a VL with an amino acid sequence as shown in SEQ ID NO: 78.

In some embodiments, the tumor cell surface antigen is selected from CD56. In some embodiments, the antibody targeted to the tumor cell surface antigen CD56 has a VH with an amino acid sequence as shown in SEQ ID NO: 79, and a VL with an amino acid sequence as shown in SEQ ID NO: 80.

In some embodiments, the tumor cell surface antigen is selected from mesothelin. In some embodiments, the antibody targeted to the tumor cell surface antigen mesothelin has a VH with an amino acid sequence as shown in SEQ ID NO: 81, and a VL with an amino acid sequence as shown in SEQ ID NO: 82.

In some embodiments, the tumor cell surface antigen is selected from HGFR. In some embodiments, the antibody targeted to the tumor cell surface antigen HGFR has a VH with an amino acid sequence as shown in SEQ ID NO: 83, and a VL with an amino acid sequence as shown in SEQ ID NO: 84.

In some embodiments, the tumor cell surface antigen is selected from Nectin-4. In some embodiments, the antibody targeted to the tumor cell surface antigen Nectin-4 has a VH with an amino acid sequence as shown in SEQ ID NO: 85, and a VL with an amino acid sequence as shown in SEQ ID NO: 86.

In some embodiments, the tumor cell surface antigen is selected from Mucin5AC. In some embodiments, the antibody targeted to the tumor cell surface antigen Mucin5AC has a VH with an amino acid sequence as shown in SEQ ID NO: 87, and a VL with an amino acid sequence as shown in SEQ ID NO: 88.

In some embodiments, the tumor cell surface antigen is selected from folate receptor α. In some embodiments, the antibody targeted to the tumor cell surface antigen folate receptor α has a VH with an amino acid sequence as shown in SEQ ID NO: 89, and a VL with an amino acid sequence as shown in SEQ ID NO: 90.

In some embodiments, the tumor cell surface antigen is carbonic anhydrase IX (CAIX). In some embodiments, the antibody targeted to the tumor cell surface antigen carbonic anhydrase IX (CAIX) has a VH with an amino acid sequence as shown in SEQ ID NO: 91, and a VL with an amino acid sequence as shown in SEQ ID NO: 92.

In some embodiments, the tumor cell surface antigen is selected from DLL1. In some embodiments, the antibody targeted to the tumor cell surface antigen DLL1 has a VH with an amino acid sequence as shown in SEQ ID NO: 93, and a VL with an amino acid sequence as shown in SEQ ID NO: 94.

In some embodiments, the tumor cell surface antigen is selected from Notch 2/3. In some embodiments, the antibody targeted to the tumor cell surface antigen Notch 2/3 has a VH with an amino acid sequence as shown in SEQ ID NO: 95, and a VL with an amino acid sequence as shown in SEQ ID NO: 96.

In some embodiments, the tumor cell surface antigen is selected from endoglin. In some embodiments, the antibody targeted to the tumor cell surface antigen endoglin has a VH with an amino acid sequence as shown in SEQ ID NO: 97, and a VL with an amino acid sequence as shown in SEQ ID NO: 98.

In some embodiments, the tumor cell surface antigen is selected from HER3. In some embodiments, the antibody targeted to the tumor cell surface antigen HER3 has a VH with an amino acid sequence as shown in SEQ ID NO: 99, and a VL with an amino acid sequence as shown in SEQ ID NO: 100.

In an aspect of the present invention, a polynucleotide is provided, and it encodes the above PD-1 mutant polypeptide or the antibody fusion protein containing the PD-1 mutant polypeptide. In some embodiments, the polynucleotide is selected from nucleotide sequences in the following group consisting of: SEQ ID NO: 1, 3, 5, 7, 9, 11, 27, 31, 51, 53, 55, 57, and 59.

In an aspect of the present invention, a vector containing the above PD-1 mutant polypeptide or the antibody fusion protein sequence containing the PD-1 mutant polypeptide is provided.

In an aspect of the present invention, a host cell is provided, and it includes the above vector.

In an aspect of the present invention, a pharmaceutical composition is provided, and it includes a pharmaceutically acceptable carrier and the above PD-1 mutant polypeptide or an antibody fusion protein containing the PD-1 mutant polypeptide.

In an aspect of the present invention, an application of the PD-1 mutant polypeptide or the antibody fusion protein containing the PD-1 mutant polypeptide as inhibitor of PD-1-PD-L1 interaction is provided.

In an aspect of the present invention, an application of the PD-1 mutant polypeptide or the antibody fusion protein containing the PD-1 mutant polypeptide as an immunomodulator is provided.

In another aspect of the present invention, an application of the PD-1 mutant polypeptide or the antibody fusion protein containing the PD-1 mutant polypeptide for treatment of tumors or cancersis provided. In some embodiments, the application for tumor or cancer treatment includes: B cell lymphoma, breast cancer, ovarian cancer, NSCLC, low-grade glioma, Hodgkin lymphoma, chronic lymphocytic leukemia (CLL), multiple myeloma, metastatic colorectal cancer, Merkel cell tumor, non-small cell lung cancer (NSCLC), squamous NSCLC, pancreatic cancer, neuromesothelioma, breast cancer, HER2-positive metastatic breast cancer, metastatic gastric cancer, liver cancer, urothelial cell carcinoma, bladder cancer, pancreatic cancer, ovarian cancer, renal cell carcinoma, multiple myeloma, soft tissue sarcoma, primary peritoneal cancer etc.

Compared with the affinity (nM) of wild-type PD-1 and PD-L1, the affinity (pM) of the high-affinity PD-1 mutant polypeptide of the present invention with PD-L1 is greatly improved. In some embodiments, the affinity of the antibody fusion protein containing the PD-1 mutant polypeptide with the recombinant PD-L1 is 39-109 times higher than that of the antibody fusion protein containing the wild-type PD-1 with the recombinant PD-L1. In some embodiments, the affinity of the antibody fusion protein containing the PD-1 mutant polypeptide with the cell surface PD-L1 is greatly enhanced relative to the affinity (almost no binding) of the antibody fusion protein containing the wild-type PD-1 with the cell surface PD-L1. Therefore, the PD-1 mutant of the present invention may be used as acompetitive inhibitor of PD-1-PD-L1 interaction. The PD-1 mutant of the present invention may also be used as animmunomodulator for, for example, the immunotherapy of tumors.

### Brief Description of the Drawings

Drawings constituting the present application are used to provide a further understanding of the present invention. The exemplary embodiments of the present invention and descriptions thereof are used to explain the present invention, and do not constitute an improper limitation to the present invention. In the drawings:
Fig. 1 is the SDS-PAGE diagram of the antibody fusion protein, herein M represents protein marker; "-" represents loading without dithiothreitol (DTT); "+" represents loading with DTT, Lane 1 is PD-1-aEGFRL, Lane 2 is PD-1m-aEGFRL, Lane 3 is aEGFR, Lane 4 is aPDL1, Lane 5 is aHER2, Lane 6 is PD-1-aHER2L, Lane 7 is PD-1m-aHER2L, Lane 8 is aGPC-3, Lane 9 is PD-1-aGPC-3L, lane 10 is PD-1m-aGPC-3L, and Lane 11 is aEGFR.
Fig. 2 shows the monomer peak of the fusion protein of PD-1 mutant polypeptide with aEGFR or aHER2 as detected by SEC.
Fig. 3 shows the ELISA binding of PD-1 mutant polypeptide and aEGFR antibody fusion protein, scaffold antibody with hEGFR.
Fig. 4 shows the ELISA binding of PD-1 mutant polypeptide and aEGFR antibody fusion protein, scaffold antibody with hHER2.
Fig. 5 shows the ELISA binding of PD-1 mutant polypeptide and aEGFR antibody fusion protein, scaffold antibody with hGPC3.
Fig. 6 shows the ELISA binding of antibody fusion proteins and control antibody with soluble hPD-L1.
Fig. 7 shows the ELISA binding of antibody fusion proteins and control antibody with soluble mPD-L1.
Fig. 8 shows binding ofantibody fusion proteins of PD-1 mutant peptide and aEGFR to cells, herein 8A is the bindingtoHepG2cells (hEGFR⁺hPDL1⁻), 8B is the binding to CHO-hPDL1cells (hEGFR⁻hPDL1⁺), 8C is the binding to MC38-hEGFRcells (hEGFR⁺mPDL1⁺), and 8D is the binding to A431 cells(hEGFR⁺hPDL1⁺).
Fig. 9 shows the binding ofantibody fusion proteins ofPD-1 mutant peptide and aGPC-3to cells, herein 9Ais the bindingtoHepG2cells (hGPC3⁺hPDL1⁻), 9B is the binding to o A431 cells (hGPC3⁻hPDL1⁺), 9C is the binding to Hepa1-6 cells (hGPC3⁻mPDL1⁺).
Fig. 10 shows that the soluble hEGFR competitively inhibits the binding ofantibodyfusion proteinsof PD-1 mutant peptide and aEGFR to cells, herein, 10A shows the binding to MC38-hEGFR cells (hEGFR⁺mPDL1⁺), 10B shows the binding to MC38-hEGFR cells (hEGFR⁺mPDL1⁺) in the presence of EGFR.
Fig.11 shows the metabolic curve of antibody fusion proteins in rats, herein 11A is the metabolic curve obtained by detecting the complete antibody fusion protein, and 11B is the metabolic curve obtained by detecting the scaffold in the antibody fusion protein.
Fig. 12 shows the efficacy antibody fusion proteins in the mouse tumor model, herein, 12A shows the tumor volume changes in mice after administration of different protein samples, and 12B shows the weight changes in mice after administration of different protein samples.

### Detailed Description of the Embodiments

The present invention is described in detail here with reference to the following definitions and embodiments. The contents of all patents and disclosures mentioned herein, including all sequences disclosed in these patents and disclosures, are explicitly incorporated herein by reference.

The term "CDR" refers to the complementarity determining region in an immunoglobulin variable region sequence. For each heavy chain and light chain variable region, there are three CDRs in each variable region of the heavy chain and the light chain, which are named as CDR1, CDR2, and CDR3. The term "CDR group" refers to a group of three CDRs that appear in a single variable region capable of binding an antigen. The exact boundaries of these CDRs are already defined according to different systems. A system described by Kabat (Kabat et al. (1987) and (1991)) not only provides a clear residue numbering system applicable to any variable regions of an antibody or binding protein, but also provides a precise definition of residue boundaries of the three CDRs in each heavy chain or light chain sequence. These CDRs may be referred to as Kabat CDRs. Certain subparts of the Kabat CDR which adopt almost the same peptide framework conformation although with great diversity at the amino acid sequence level are named as L1, L2 and L3 or H1, H2 and H3 by Chothia and colleagues (Chothia and Lesk (1987) J. Mol. Biol. 196:901-917; and Chothia et al. (1989) Nature 342:877-883), herein "L" and "H" refer to light chain and heavy chain regions, respectively. These regions may be referred to as Chothia CDR, with a boundary overlapped with the Kabat CDR. Other boundaries defining CDR that overlapped with the Kabat CDR are described by Padlan (1995) FASEB J. 9: 133-139 and MacCallum (1996) J. Mol. Biol. 262(5): 732-45). There are other CDR boundary definitions that may not strictly follow one of the systems in this article, but still overlap with Kabat CDR. These CDRs may be shortened or lengthen based on the prediction or experiment in which specific residues or a residue group or even the entire CDR does not significantly affect the antigen binding. This article uses the Kabat's CDR definition (see http://www.bioinf.org.uk/abs/ for details).

The term "tumor cell surface antigen" of the present invention refers to an antigen that is expressed on the surface of a tumor cell and may be targeted for disease treatment. In certain embodiments, the targeted antigen is selected from the antigen that is preferentially expressed on the surface of tumor cells (such as solid tumors or hematological tumor cells): non-limiting examples of specific tumor-associated antigen include, for example, EGFR, HER2, HER3, HER4, GPC3, PSMA, CD27, CD33, CD37, CD38, CD51, CD56, mesothelin, HGFR, Nectin-4, Mucin 5AC, folate receptor α, carbonic anhydrase IX (CAIX), DLL1, Notch2, Notch3, endoglin, MUC1, MUC2, MUC3A, MUC3B, MUC4, MUC5B, MUC6, MUC7, MUC8, MUC12, MUC13, MUC15, MUC16, MUC17, MUC19, MUC20, VEGFR-1 (FLT1), VEGFR-2 (KDR/FIK-1), VEGFR-3, PDGF-RA, PDGF-RB, IGF-1R, IGF2B3, K-RAS, N-RAS, BAFF-R, EpCAM, SAGE, XAGE-1b, BAGE, MAGE protein (such as MAGE-1, MAGE-2, MAGE-3. MAGE-4, MAGE-6, MAGE-9, MAGE-10, MAGE-12), GAGE-1, GAGE-2, GAGE-8, GAGE-3, GAGE-4, GAGE-5, GAGE-6 , GAGE-7, XAGE-1b/GAGED2a, RAGE-1, RBAF600, CD-11α, CD16A, CD21, CD22, dipeptidyl-peptidase 4 (CD26), CD30, CD32B, CD45, CD52, CD70, CD80, CD60, CD62 , CD72, CD79a, CD79B, CD123, Ly6D, Ly6E, Ly6K, gp100/Pmel17, EDAR, GFRA1 (GDNF-Ra1), MRP4, RET, STEAP1, STEAP2, TENB2, E16 (LAT1, SLC7A5), SLC35D3, MPF, SCL34A2 , Sema 5b, PSCAhIg, ETBR, MSG783, FcRH1, FcRH2, NCA, MDP, IL20Ra, EphA2, EphA3, EphB2R, ASLG659, GEDA, CXCR5, P2X5, LY64, IRTA2, TMEF1, TMEM46, TMEM118, LGR5, GPR19, GPR172 CLL1, RNF43, KISS1R, ASPHD1, CX ORF61, HAVCR1, epiregulin, amphiregulin, lipophilin, AIM-2, ALDH1A1, a-actinin-4, ARTC1, BING-4, CALCA, CASP-5, CASP-8, cdc27, CDK4 , CDKN2A, CLPP, COA-1, CPSF, Cw6, RANKL, DEK-CAN, DKK1, EFTUD2, elongation factor 2, ENAH (hMena), ETV6-AML1, EZH2, FLT3-ITD, FN1, G250, MN, CAIX , GnTVf, GPNMB, HERV-K-MEL, hsp70-2, IDO1, IL13Ra2, KIF20A, KK-LC-1, KM-HN-1, LAGE-1, Lengsin, M-CSF, MART-1, Melan- A/MART-1, MART2, MCSP, mdm-2, ME-1, Meloe, MMP-2, MMP-7, mucin, MUM-1, MUM-2, MUM-3, myosin I, NA88 -A, PAP, neo-PAP, NFYC, NY-BR1, NY-BR62, NY-BR85, NY-ESO1, NY-ESO-1/LAGE-2, RAB38/NY-MEL-1, OA1, OGT, OS -9, p53, PAX3, PAX5, PBF, PML-RARa, PRAME, PRDX5, PSMA (FOLH1), PTPRK, RGS5, Rho, RhoC, RNF43, RU2AS, protein isolate 1, SIRT2, SNRPD1, SOX10, Sp17, SSX-2, SSX-4, survivin, SYT-SSX1 or -SSX2, TAG-1, TAG-2, telomerase, TGF-β, TGF-beta RII, TRAG-3, TRP-2, TRP2-INT2, VEGF, WT1, TRPM4, CRIPTO, glycoprotein IIb/IIIa receptor, glycolipid GD2, GD3, folate receptor 1 (FOLR1), IFNy, IFNα,β,ω receptor 1, TROP-2, Glycoprotein NMB, MMP9, GM3, mesothelin, fibronectin extra-domain B, endoglin, Rhesus D, plasma kallikrein, CS, thymic stromal lymphopoietin, mucosal addressin cell adhesion molecule, nectin 4. NGcGM3, DLL3, DLL4, CLEC12A, KLB, FGFR1C, CEA, BCMA, p-cadherin, FAP, DR1, DR5, DR13, PLK, B7-H3, c-Met, gpA33, gp100/Pmel17, gp100, TRP-1/gp75, BCR-ABL, AFP, ALK, β-chain protein, BRCA1, BORIS, CA9, Caspase-8, CDK4, CTLA4, Cyclin-B1, Cyclin D1, Cyclin-A1 CYP1B1, Fra-1, GloboH, Glypican-3, GM3, HLA/B-RAF, hTERT, LMP2, mesothelin, ML-IAP, NA17, OX40, p15, PPLR, PCTA-1, PLAC1, PRLR, PRAME, SART-1, SART-3, TAG-72, TMPRSS2, Tn, tyrosinase and urinary plaque protein-3. CCR1, CCR2, CCR3, CCR4, CCR5, CCR6, CCR7, CCR8, CCR9, CCR10, CCL27, CCL28, CX3CR1, CXCR1, CXCR2, CXCR3, CXCR4, CXCR5, CXCR6, androgen receptor (AR), calcitriol receptor (CR), estrogen receptor (ER), corticotropin releasing hormone receptor (CRHR), glucagon receptor (GCGR), gonadotropin receptor (FSHR, LHR), or melanocortin 1 receptor (MC1R, MSHR).

### Embodiment

It should be noted that, in the case without conflict, the embodiments in the present application are merely examples for illustration, and are not intended to limit the present invention in any forms.

### Embodiments

### Embodiment 1: Construction of eukaryotic expression vector of antibody fusion protein

PCR amplification of anti-human EGFR antibody heavy chain variable region (aEGFR VH) (1-357 bp of SEQ ID No. 3), EGFR antibody light chain variable region (aEGFR VL) (1-321 bp of SEQ ID NO. 5), anti-human HER2 antibody heavy chain variable region (aHER2 VH) (1-360 bp of SEQ ID No. 9), anti-human HER2 antibody light chain variable region (aHER2 VL) (1-321 bp of SEQ ID NO. 7), anti-human GPC-3 antibody heavy chain variable region (aGPC-3 VH) (SEQ ID NO.41), anti-human GPC-3 antibody light chain variable region (aGPC-3 VL) (SEQ ID NO.43), and human PD-1 mutant polypeptide PD-1m (SEQ ID No. 1) genes were performed. The amplified aEGFR VH and aHER2 VH genes were cloned into the pFuse-hIgG1-Fc2 vector (InvivoGen) (herein 9 mutations contained in hIgG1-Fc on the vector: E233P, L234V, L235A, ΔG236, A327G, A330S, P331S, E356D, and M358L are all completed by our laboratory), and aEGFR VL and aHER2 VL were cloned into the pFuse2-CLIg-Hk vector (InvivoGen) by enzyme digestion and ligation. the amplified PD-1m gene was cloned into the N-terminal of a EGFR antibody light chain or the N-terminal of aHER2 antibody light chain constructed as described above by enzyme digestion and ligation. All constructed vectors were verified by sequencing.

**Table 1: Sequence listing**

| Construct | Nucleotide sequence | Amino acid sequence |
|---|---|---|
| | Seq ID No: | Seq ID No: |
| PD-1m | 1 | 2 |
| aEGFR HC | 3 | 4 |
| aEGFR LC | 5 | 6 |
| aHER2 LC | 7 | 8 |
| aHER2 HC | 9 | 10 |
| PD-1 | 11 | 12 |
| aEGFR CDR1-H | / | 13 |
| aEGFR CDR2-H | / | 14 |
| aEGFR CDR3-H | / | 15 |
| aEGFR CDR1-L | / | 16 |
| aEGFR CDR2-L | / | 17 |
| aEGFR CDR3-L | / | 18 |
| aHER2 CDR1-H | / | 19 |
| aHER2 CDR2-H | / | 20 |
| aHER2 CDR3-H | / | 21 |
| aHER2 CDR1-L | / | 22 |
| aHER2 CDR2-L | / | 23 |
| aHER2 CDR3-L | / | 24 |
| PD-1-aEGFR-LC | 25 | 26 |
| PD-1m-aEGFR-LC | 27 | 28 |
| PD-1-aHER2-LC | 29 | 30 |
| PD-1m-aHER2-LC | 31 | 32 |
| aEGFR VH | 33 | 34 |
| aEGFR VL | 35 | 36 |
| aHER2 VH | 37 | 38 |
| aHER2 VL | 39 | 40 |
| aGPC3 VH | 41 | 42 |
| aGPC3 VL | 43 | 44 |
| aGPC3 CDR1-H | | 45 |
| aGPC3 CDR2-H | | 46 |
| aGPC3 CDR3-H | | 47 |
| aGPC3 CDR1-L | | 48 |
| aGPC3 CDR2-L | | 49 |
| aGPC3 CDR3-L | | 50 |
| PD-1m-aGPC-3 HC | 51 | 52 |
| PD-1m-aGPC-3 LC | 53 | 54 |
| aGPC-3 LC-PD-1m | 55 | 56 |
| aGPC-3 HC | 57 | 58 |
| aGPC-3 LC | 59 | 60 |
| aPSMAVH | 61 | 62 |
| aPSMA VL | 63 | 64 |
| aEGFR VH-2 | | 65 |
| aEGFR VL-2 | | 66 |
| aHER2 VH-2 | | 67 |
| aHER2 VL-2 | | 68 |
| aCD27 VH | | 69 |
| aCD27 VL | | 70 |
| aCD33 VH | | 71 |
| aCD33 VL | | 72 |
| aCD37 VH | | 73 |
| aCD37 VL | | 74 |
| aCD38 VH | | 75 |
| aCD38 VL | | 76 |
| aCD51 VH | | 77 |
| aCD51 VL | | 78 |
| aCD56 VH | | 79 |
| aCD56 VL | | 80 |
| anti-mesothelin VH | | 81 |
| anti-mesothelin VL | | 82 |
| aHGFR VH | | 83 |
| aHGFR VL | | 84 |
| anti-nectin 4 VH | | 85 |
| anti-nectin 4 VL | | 86 |
| anti-Mucin 5AC VH | | 87 |
| anti-Mucin 5AC VH | | 88 |
| anti-folate receptor α VH | | 89 |
| anti-folate receptor α VL | | 90 |
| anti-CAIX VH | | 91 |
| anti-CAIX VL | | 92 |
| anti-DLL1 VH | | 93 |
| anti-DLL1 VH | | 94 |
| anti-Notch2/3 VH | | 95 |
| anti-Notch2/3 VL | | 96 |
| anti-endoglin VH | | 97 |
| anti-endoglin VL | | 98 |

| | | |
|---|---|---|
| Note: PD-1: Human PD-1 PD-1m: Human PD-1 mutant | | |

### Embodiment 2: Expression, purification and SEC detection of antibody fusion protein

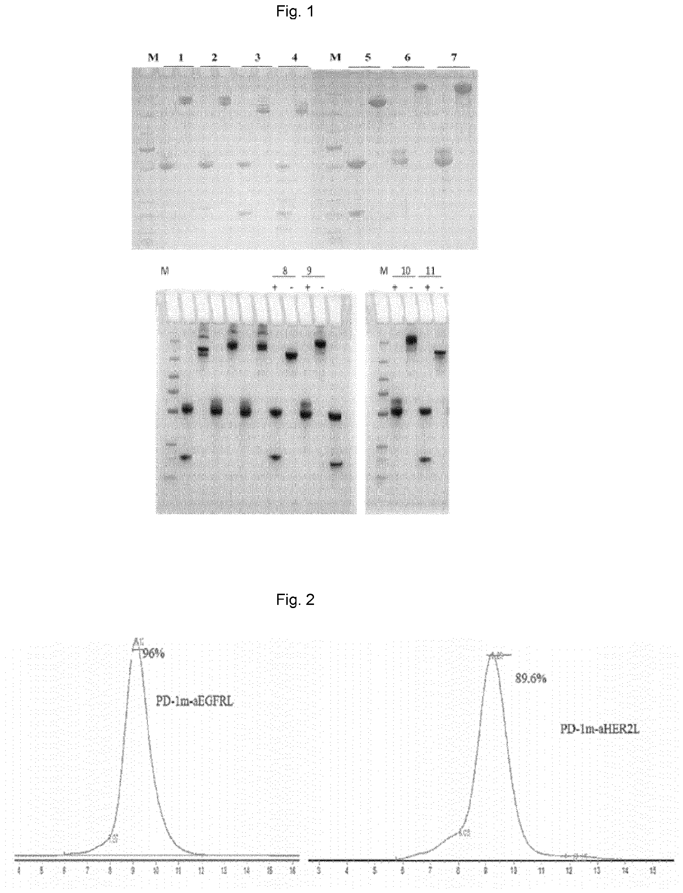

The heavy chain and light chain of the fusion protein expression vector constructed in Embodiment 1 were transiently transfected into FreeStyle HEK293 cells (ThermoFisher), and the amount of the plasmid for the heavy chain and the plasmid for the light chain during transfection is a molar ratio of 1:1. 28 ml of FreeStyle HEK 293 (3×10⁷ cells/ml) were seeded in a 125 ml cell culture flask, plasmids were diluted with 1 ml of Opti-MEM (Invitrogen), and then added to 1 ml of Opti-MEM containing 60 µl 293Fectin (Invitrogen). After standing at room temperature for 30 min, the plasmid-293Fectin mixture was added to the cell culture medium, and incubated at 125rpm, 37°C and 5% CO₂. Cell culture supernatant was collected at 96 h after transfection, and purified by Protein A Resin (Genscript). The purified proteins were applied to SDS-PAGE. Results are shown in Fig. 1, indicating that the antibody fusion protein with expected molecular weight was successfully expressed.

The antibody fusion protein purified by Protein A resin was analyzed by size exclusion chromatography by GE's AKTA .The column used was: Superdex 200 Increase 10/300 GL size exclusion chromatography column, and the solution used was PBS buffer (0.010 M phosphate buffer, 0.0027 M KCl, 0.14 M NaCl, pH 7.4). It is seen from the chromatogram in Fig. 2 that the expression of the antibody fusion protein of PD-1 and aEGFR or aHER2 has considerable purity.

### Embodiment 4: Analysis of in vitro activity of antibody fusion protein

### 4.1 Analysis of Binding to human EGFR

hEGFR-hIGg1Fc (SinoBiological) (100 ng/well) was coated in a 96-well plate, and incubated overnight at 4°C. After blocked with PBST (0.5% Tween-20 in PBS) containing 2% skimmed milk powder for 1 hour at room temperature, the gradiently diluted antibody (aEGFR) fusion proteins (10pM-1.2nM) were added and incubated at room temperature for 2 h. After being washed with PBST containing 2% skimmed milk powder for 4-5 times, anti-human kappa light chain-HRP (Sigma A7146, 1:3000) secondary antibody was added and incubated at room temperature for 1 h, and then washed with PBST containing 2% skimmed milk powder for 4-5 times. Color development was performed by QuantaBlu fluorescent peroxidase substrate (Life technologies, Cat.15169) and read at 325 nm and 420 nm, or by TMB color reagent (BioLegend , Cat. 421101) and read at 650 nm (unterminated) or 450 nm (terminated). Data was analyzed by nonlinear regression using specific binding model with Prizm Graphpad software

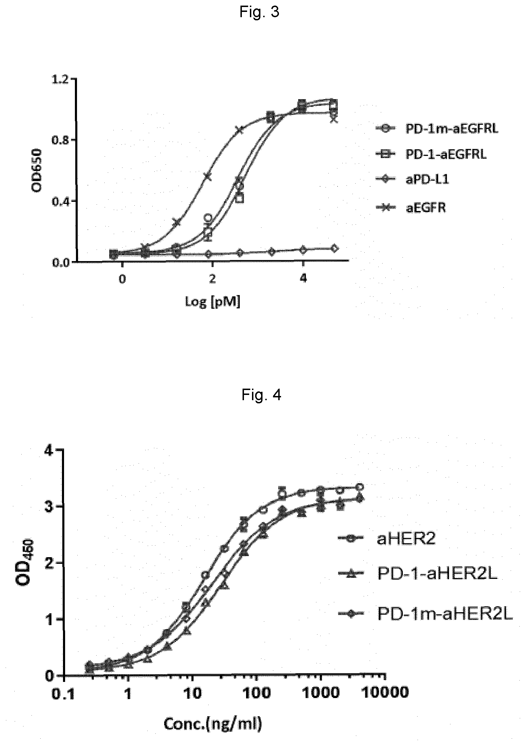

As shown in Fig. 3, the antibody fusion proteins PD-1m-aEGFR and PD-1-aEGFR have similar affinities to EGFR.

### 4.2 Analysis of Binding to human HER2

hHER2-His (Aero) (100 ng/well) was coated in a 96-well plate, and incubated overnight at 4°C. After blocked with PBST (0.5% Tween-20 in PBS)containing 2% skimmed milk powder for 1 hour at room temperature, the gradiently diluted antibody (aHER2) fusion proteins (10pM-1.2nM) were added and incubated at room temperature for 2 h. After being washed with PBST containing 2% skimmed milk powder for 4-5 times, anti-human kappa light chain-HRP (Sigma A7146, 1:3000) secondary antibody was added and incubated at room temperature for 1 h, and then washed with PBST containing 2% skimmed milk powder for 4-5 times. Color development was performed by QuantaBlu fluorescent peroxidase substrate (Life technologies, Cat.15169) and read at 325 nm and 420 nm, or by TMB color reagent (BioLegend , Cat. 421101) and read at 650 nm (unterminated) or 450 nm (terminated). Data was analyzed by nonlinear regression using specific binding model with Prizm Graphpad software.

As shown in Fig. 4, PD-1-aHER2 and PD-1m-aHER2 have similar affinities to HER2. Compared with the positive control aHER2, the fusion of PD-1 or PD-1m has little effect on the binding of the scaffold antibody to HER2.

### 4.3 Analysis of binding to human GPC-3

hGPC-3 (BioLegend) (100 ng/well) was coated in a 96-well plate, and incubated overnight at 4°C. After blocked with PBST(0.5% Tween-20 in PBS) containing 2% skimmed milk powder for 1 h at room temperature, the antibody (aGPC-3) fusion proteins in gradient dilutions (25pM-3nM) were added and incubated at room temperature for 2 h. After being washed with PBST containing 2% skimmed milk powder for 4-5 times, anti-human kappa light chain-HRP (Sigma A7146, 1:3000) secondary antibody was added and incubated at room temperature for 1 h, and then washed with PBST containing 2% skimmed milk powder for 4-5 times. Color development was performed , by QuantaBlu fluorescent peroxidase substrate (Life technologies, Cat.15169) and read at 325 nm and 420 nm, or by TMB color reagent (BioLegend , Cat. 421101) and read at 650 nm (unterminated) or 450 nm (terminated). Data was analyzed by nonlinear regression using specific binding model with Prizm Graphpad software.

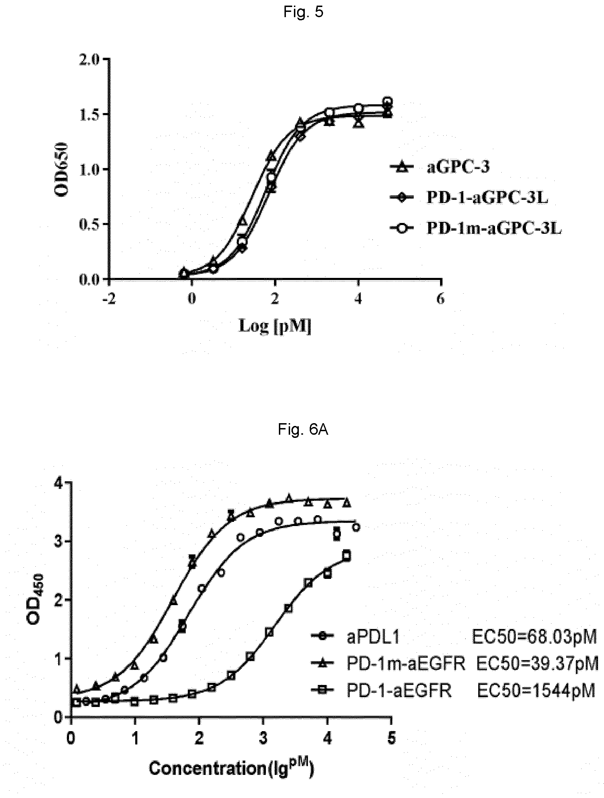

As shown in Fig. 5, the affinities of PD-1-aGPC-3 and PD-1m-aGPC-3 to GPC3 are similar to the affinity of scaffold antibody aGPC-3, indicating that the fusion of PD-1 or PD-1m has little effect on the binding of scaffold antibody to GPC-3.

### 4.4 Analysis of binding to human PD-L1 or mouse PD-L1

hPD-L1-hIGg1Fc (SinoBiological) or mouse PD-L1-Fc (SinoBiological) (100ng/well) was coated in 96-well plates, and incubated overnight at 4°C. After blocked with PBST(0.5% Tween-20 in PBS) containing 2% skimmed milk powder at room temperature for 1 h, the antibody fusion protein in gradient dilutions (25pM-3nM) was added and incubated at room temperature for 2 h. After being washed with PBST containing 2% skimmed milk powder for 4-5 times, anti-human kappa light chain-HRP (Sigma A7146, 1:3000) secondary antibody was added and incubated at room temperature for 1 h, and then washed with PBST containing 2% skimmed milk powder for 4-5 times. Colordevelopment was performed by QuantaBlu fluorescent peroxidase substrate (Life technologies, Cat.15169) and read at 325 nm and 420 nm, or by TMB color reagent (BioLegend , Cat. 421101) and read at 650 nm (unterminated) or 450 nm (terminated). Data was analyzed by nonlinear regression using specific binding model with Prizm Graphpad software.

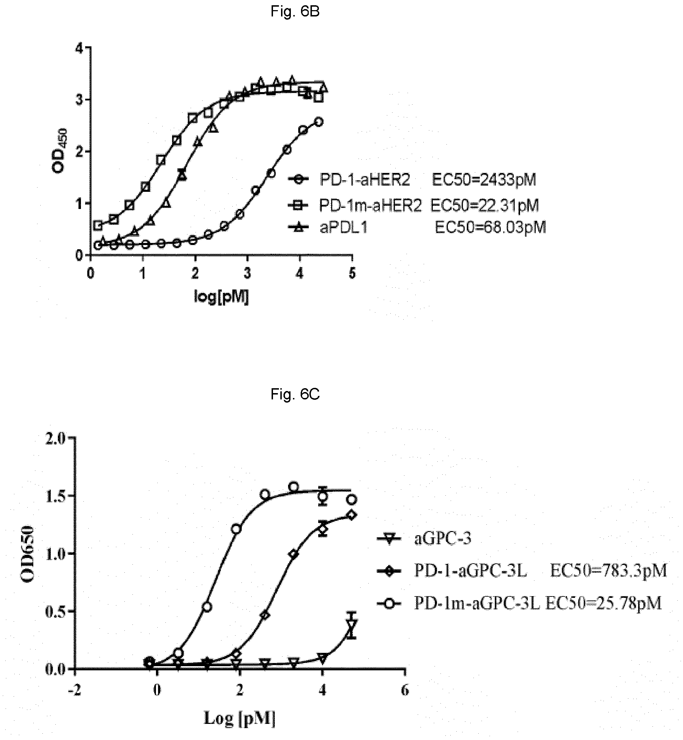

As shown in Fig. 6, the affinity of PD-1m antibody fusion protein (PD-1m-aEGFRL, PD-1m-aHER2L or PD-1m-aGPC-3L) to human PD-L1 is similar to that of the positive control aPD-L1 to human PD-L1, and is much higher than that of PD-1 antibody fusion protein to human PD-L1 (25-35 times).

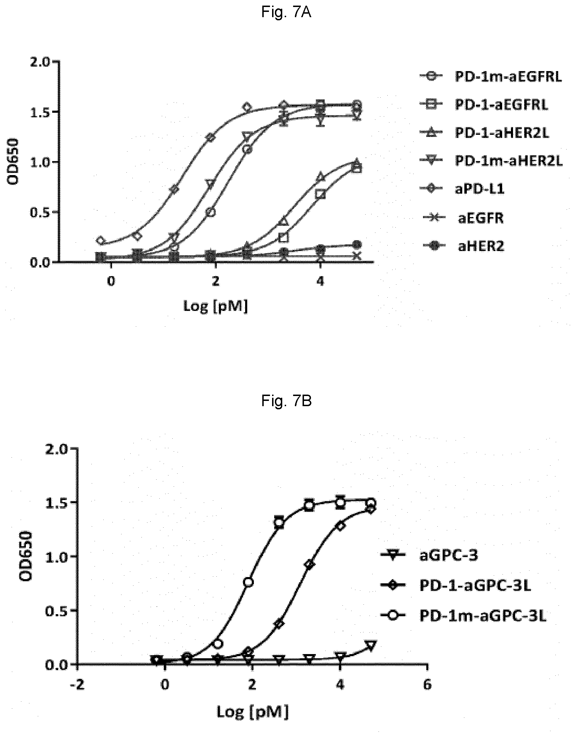

As shown in Fig. 7, the affinity of PD-1m antibody fusion protein (PD-1m-aEGFRL, PD-1m-aHER2L or PD-1m-aGPC-3L) to mouse PD-L1 is similar to that of the positive control aPD-L1 to mouse PD-L1, and is much higher than that of PD-1 antibody fusion protein to mouse PD-L1.

### 4.5Flow cytometry analysis on binding of antibody fusion protein to cells

A431, MC38-hEGFR, CHO-PD-L1, HepG2, and Hepa1-6 cells (DMEM medium containing 10% FBS, 1% double antibody) were cultured. After trypsinization, 2*10⁵ cells were taken and washed with pre-cooled PBS for 3 times. After blocked with PBS containing 2% FBS at 4°C, supernatant was discarded by centrifugation, 100 pL of the antibody fusion proteins with different concentrations diluted by the PBS solution containing 2% FBS were added and incubated at 4°C for 1 h. The unbound antibody fusion protein was washed off with PBS containing 2% FBS, and secondary antibody Mouse Anti-Human IgG Fc-APC (Southern biotech) was added and incubated at 4°C for 1 h. After being washed with PBS containing 2% FBS for three times, the fluorescence intensity was detected by flow cytometry.

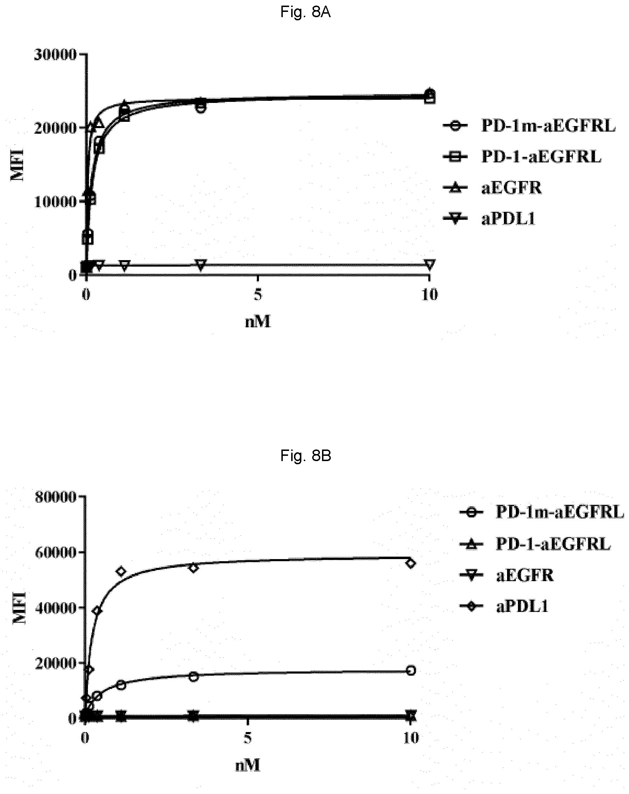

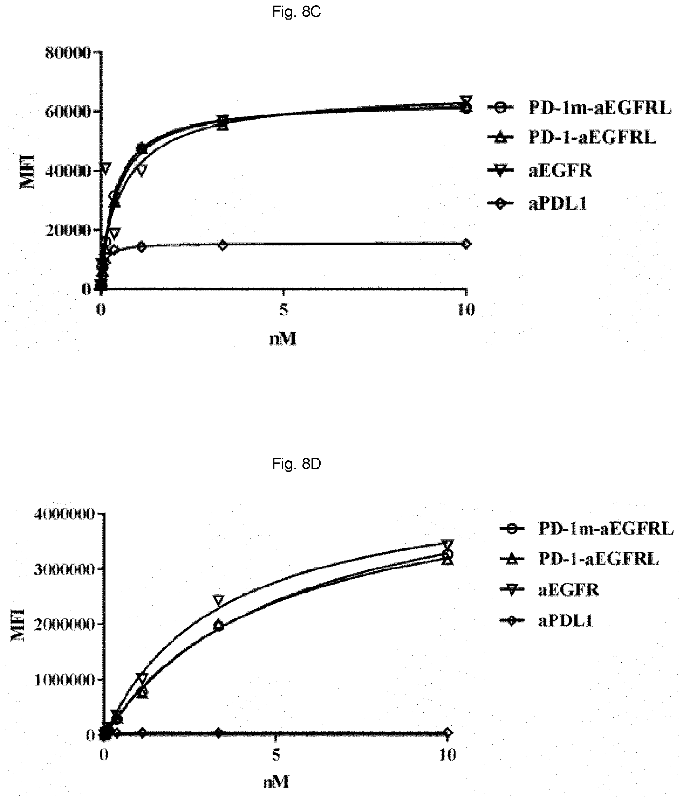

Results of the binding of PD-1m-aEGFRL to cells are shown in Fig. 8. Compared with aEGFR, the binding of PD-1m-aEGFRL and PD-1-aEGFRL to HepG2 cell surface EGFR is very similar with a slight reduction(Fig. 8A), which is consistent with ELISA results. Compared with PD-1-aEGFRL, PD-1m-aEGFRL can bind to hPD-L1 on the surface of CHO-hPD-L1 cells, and the binding activity is similar to that of aPD-L1 (Fig. 8B), which is consistent with ELISA results. Compared with aEGFR, PD-1m-aEGFRL or PD-1-aEGFRL has slightly better cell binding activity to MC38-hEGFR (Fig. 8C), indicating a synergistic effect.

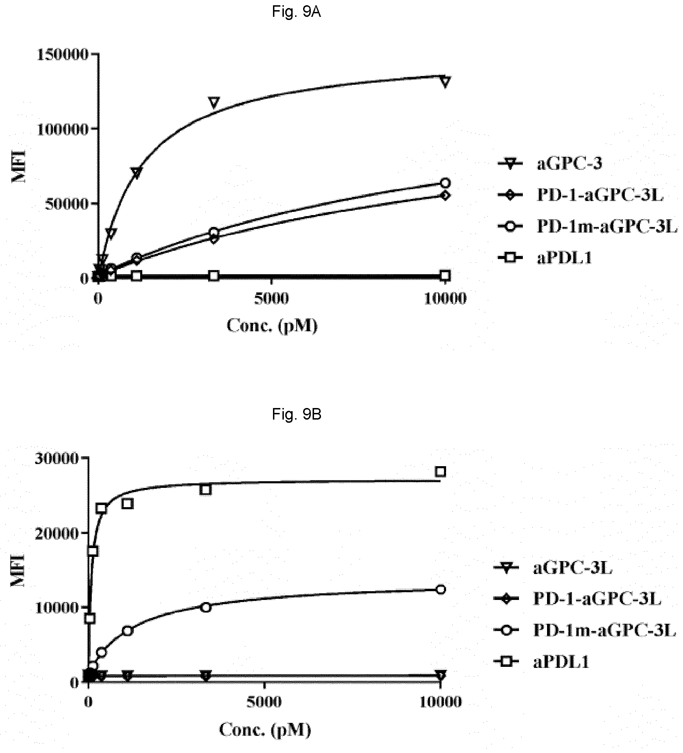

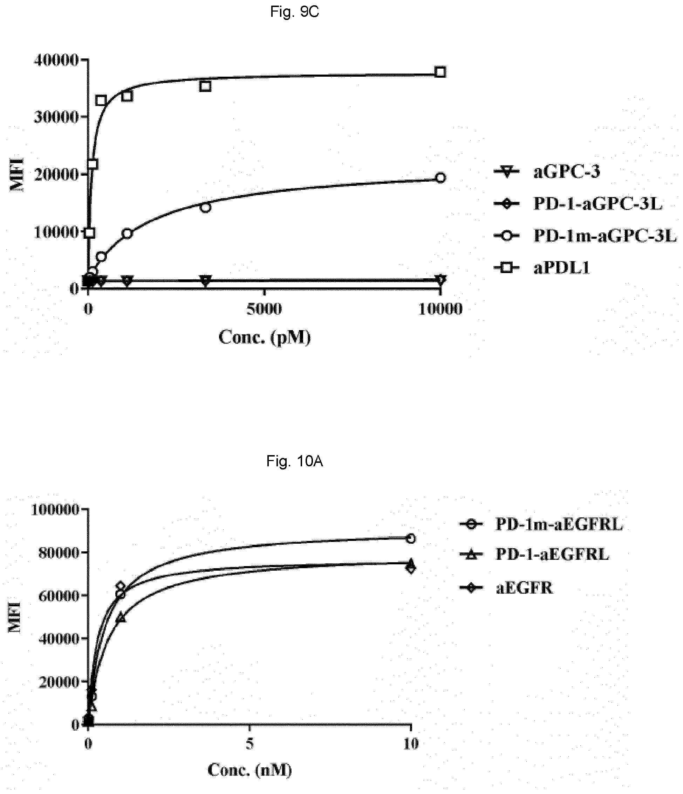

Results of the binding of PD-1m-aGPC-3L to cells are shown in Fig. 9. Compared with aGPC-3, the binding of PD-1m-a GPC-3L and PD-1-aGPC-3L to HepG2 cell surface GPC-3 is very similar with a slight reduction(Fig. 9A). Compared with PD-1-aGPC-3L, PD-1m-aGPC-3L can bind to hPD-L1 (9B) on the surface of A431 cells and mPDL1 (9C) on the surface of Hepa1-6 cells.

### 4.6 Competitive inhibition test

MC38 cells expressing human EGFR (MC38-hEGFR, constructed by our laboratory) (DMEM medium containing 10% FBS, 1% double antibody) were cultured. After trypsinization, 2*10⁵ cells were taken and washed with pre-cooled PBS for 3 times. After being blocked with PBS containing 2% FBS at 4°C, supernatant was discarded by centrifugation, 100 pL of the antibody fusion proteins with different concentrations diluted by the PBS solution containing 2% FBS were added and incubated at 4°C for 1 h. The unbound antibody fusion protein was washed off with PBS containing 2% FBS, and secondary antibody Mouse Anti-Human IgG Fc-APC (Southern biotech) was added and incubated at 4°C for 1 h. After being washed with PBS containing 2% FBS for three times, the fluorescence intensity was detected by flow cytometry.

After being washed with PBS for 3 times, supernatant was discarded by centrifugation, and the PBS solution containing 2% FBS was added and incubated at 4°C for 1 h. After the incubation, the supernatant was discarded by centrifugation, and 100 pL of antibody fusion proteins with different concentrations diluted by PBS containing 2% FBS was added for cell binding, or the antibody fusion proteins with the different concentrations were incubated with 500 nM of EGFR-His and cells for competitive binding. The unbound antibody fusion protein was washed off with PBS containing 2% FBS, a secondary antibody Mouse Anti-Human IgG Fc-APC (southern biotech) is added and incubated at 4°C for 1 h. After being washed with PBS containing 2% FBS for three times, the fluorescence intensity was detected by flow cytometry.

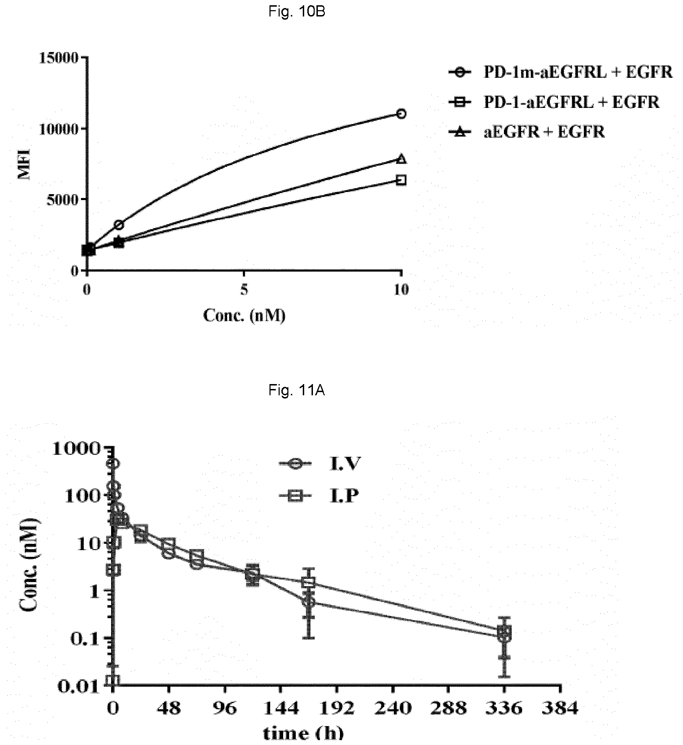

Results are shown in Fig. 10. The binding of PD-1m-aEGFRL to MC38-hEGFR cells can be weakened by free hEGFR-his in the solution, but the magnitude of is less than that of the binding ability of the positive control aEGFR or PD-1-aEGFR to the MC38-hEGFR cells.

### 4.7 PK study in rats

The antibody fusion protein was intraperitoneal-injected (I.P.) or tail-vein-injected (I.V.) into SD male rats (3 rats/group). Heparin anticoagulant blood was collected from the tail vein at 15 min, 30 min, 1h, 4h, 8h, 1d, 2d, 3d, 5d, 7d and 14d after injection. After centrifugation, plasma was collected, and stored at -80°C for further use. The antibody fusion protein and scaffold antibody in the plasma was performed with reference to Embodiment 4.1.

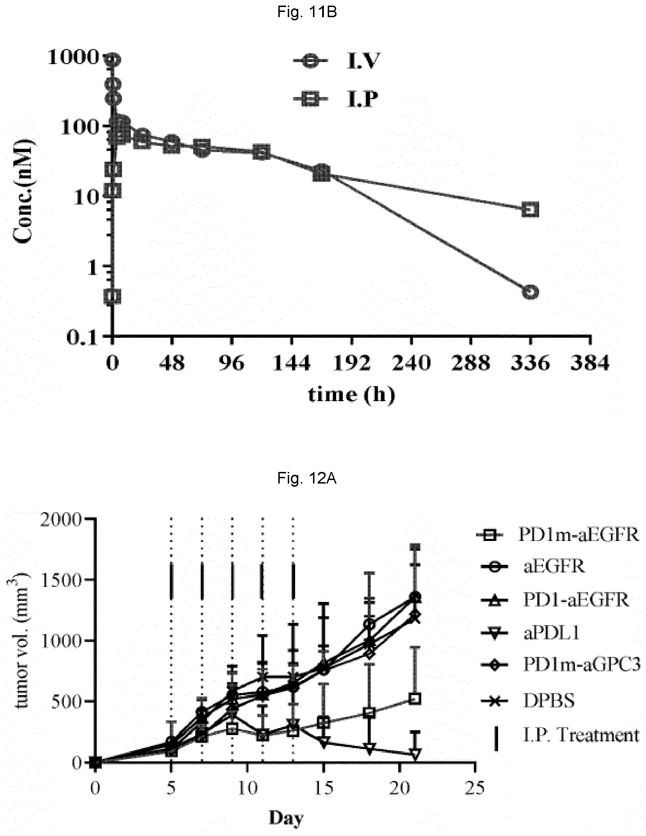

As shown in Fig. 11, the scaffold of the antibody fusion protein has a longer half-life than that of the antibody fusion protein.

### 4.8 In vivo efficacy

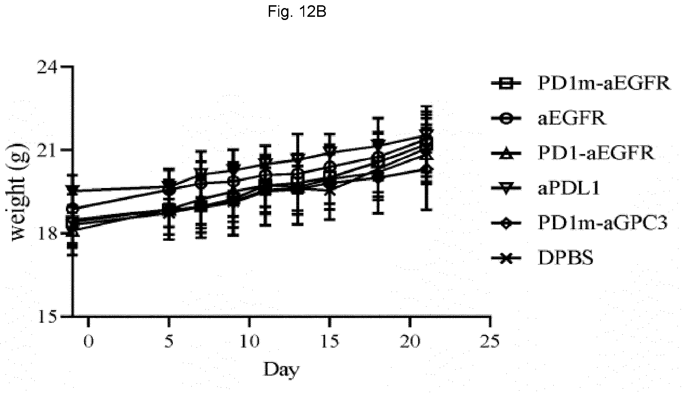

The ability of PD1m-aEGFR to inhibit tumor in tumor-bearing mice was performed on C57BL/6 mice. 5×10⁶/100ul MC38-hEGFR cells were resuspended in a 200 pL serum-free medium and then subcutaneously injected to the mice (Day 0). The treatment or control administration was started while the tumor size was 50-200 mm³, and tumor and body weight was recorded. The frequency of the administration is indicated by an arrow in Fig. 12, and the size of the tumor mass in the experimental mice is measured with a caliper. The tumor volume is calculated as follows: tumor volume=width*width*length/2 .

As shown in Fig. 12, the antibody fusion protein can effectively inhibit tumor growth without significant side effects such as weight loss. The control bispecific antibody(PD1m-aGPC3) without EGFR targeting cannot inhibit tumor growth at all, proving that the tumor-targeted PD1m-aEGFR has good selectivity and safety.

## Claims

1. A PD-1 mutant polypeptide, wherein the mutant polypeptide has the amino acid sequence of SEQ ID NO: 2.

2. An antibody fusion protein comprising the PD-1 mutant polypeptide of claim 1.

3. The antibody fusion protein of claim 2, wherein the fusion protein comprises:
a) the PD-1 mutant polypeptide;
b) an antibody targeted to a tumor cell surface antigen, wherein the tumor cell surface antigen is selected from EGFR, HER2, HER3, GPC3, PSMA, CD27, CD33, CD37, CD38, CD51, CD56, mesothelin, HGFR, Nectin-4, Mucin 5AC, folate receptor α, carbonic anhydrase IX (CAIX), DLL1, Notch2, Notch3, and endoglin.

4. The antibody fusion protein of claim 3, wherein the antibody targeting to the tumor cell surface antigen EGFR has: sequences HCDR1, HCDR2 and HCDR3 as shown in SEQ ID NO: 13, SEQ ID NO: 14, and SEQ ID NO: 15, and sequences LCDR1, LCDR2 and LCDR3 as shown in SEQ ID NO: 16, SEQ ID NO: 17, and SEQ ID NO: 18; or a heavy chain variable region (VH) with an amino acid sequence as shown in SEQ ID NO: 34, and a light chain variable region (VL) with an amino acid sequence as shown in SEQ ID NO: 36.

5. The antibody fusion protein of claim 3, wherein the antibody targeted to the tumor cell surface antigen EGFR has a heavy chain variable region (VH) with an amino acid sequence as shown in SEQ ID NO: 65, and a light chain variable region (VH) with an amino acid sequence as shown in SEQ ID NO: 66.

6. The antibody fusion protein of claim 3, wherein the antibody targeted to the tumor cell surface antigen HER2 has sequences HCDR1, HCDR2 and HCDR3 as shown in SEQ ID NO: 19, SEQ ID NO: 20, and SEQ ID NO: 21, and sequences LCDR1, LCDR2 and LCDR3 as shown in SEQ ID NO: 22, SEQ ID NO: 23, and SEQ ID NO: 24; or a VH with an amino acid sequence as shown in SEQ ID NO: 38, and a VL with an amino acid sequence as shown in SEQ ID NO: 40.

7. The antibody fusion protein of claim 3, wherein the antibody targeted to the tumor cell surface antigen HER2 has a VH with an amino acid sequence as shown in SEQ ID NO: 67, and a VL with an amino acid sequence as shown in SEQ ID NO: 68.

8. The antibody fusion protein of claim 3, wherein the antibody targeted to the tumor cell surface antigen GPC3 has sequences HCDR1, HCDR2 and HCDR3 as shown in SEQ ID NO: 45, SEQ ID NO: 46, and SEQ ID NO: 47, and sequences LCDR1, LCDR2, and LCDR3 as shown in SEQ ID NO: 48, SEQ ID NO: 49, and SEQ ID NO: 50; or a VH with an amino acid sequence as shown in SEQ ID NO: 42, and a VL with an amino acid sequence as shown in SEQ ID NO: 44.

9. The antibody fusion protein of claim 3, wherein the antibody targeting to the tumor cell surface antigen PSMA has a VH with an amino acid sequence as shown in SEQ ID NO: 62, and a VL with an amino acid sequence as shown in SEQ ID NO: 64.

10. The antibody fusion protein of claim 3, wherein the antibody targeted to the tumor cell surface antigen CD27 has a VH of an amino acid sequence as shown in SEQ ID NO: 69, and a VL of an amino acid sequence as shown in SEQ ID NO: 70.

11. The antibody fusion protein of claim 3, wherein the antibody targeted to the tumor cell surface antigen CD33 has a VH of an amino acid sequence as shown in SEQ ID NO: 71, and a VL of an amino acid sequence as shown in SEQ ID NO: 72.

12. The antibody fusion protein of claim 3, wherein the antibody targeted to the tumor cell surface antigen CD37 has a VH of an amino acid sequence as shown in SEQ ID NO: 73, and a VL of an amino acid sequence as shown in SEQ ID NO: 74.

13. The antibody fusion protein of claim 3, wherein the antibody targeted to the tumor cell surface antigen CD38 has a VH of an amino acid sequence as shown in SEQ ID NO: 75, and a VL of an amino acid sequence as shown in SEQ ID NO: 76.

14. The antibody fusion protein of claim 3, wherein the antibody targeted to the tumor cell surface antigen CD51 has a VH of an amino acid sequence as shown in SEQ ID NO: 77, and a VL of an amino acid sequence as shown in SEQ ID NO: 78.

15. The antibody fusion protein of claim 3, wherein the antibody targeted to the tumor cell surface antigen CD56 has a VH of an amino acid sequence as shown in SEQ ID NO: 79, and a VL of an amino acid sequence as shown in SEQ ID NO: 80.

16. The antibody fusion protein of claim 3, wherein the antibody targeting to the tumor cell surface antigen mesothelin has a VH with an amino acid sequence as shown in SEQ ID NO: 81, and a VL with an amino acid sequence as shown in SEQ ID NO: 82.

17. The antibody fusion protein of claim 3, wherein the antibody targeted to the tumor cell surface antigen HGFR has a VH of an amino acid sequence as shown in SEQ ID NO: 83, and a VL of an amino acid sequence as shown in SEQ ID NO: 84.

18. The antibody fusion protein of claim 3, wherein the antibody targeted to the tumor cell surface antigen Nectin-4 has a VH of an amino acid sequence as shown in SEQ ID NO: 85, and a VL of an amino acid sequence as shown in SEQ ID NO: 86.

19. The antibody fusion protein of claim 3, wherein the antibody targeted to the tumor cell surface antigen Mucin 5AC has a VH with an amino acid sequence as shown in SEQ ID NO: 87, and a VL with an amino acid sequence as shown in SEQ ID NO: 88.

20. The antibody fusion protein of claim 3, wherein the antibody targeted to the tumor cell surface antigen folate receptorahas a VH of an amino acid sequence as shown in SEQ ID NO: 89, and a VL of an amino acid sequence as shown in SEQ ID NO: 90.

21. The antibody fusion protein of claim 3, wherein the antibody targeting to the tumor cell surface antigen carbonic anhydrase IX (CAIX) has a VH with an amino acid sequence as shown in SEQ ID NO: 91, and a VL with an amino acid sequence as shown in SEQ ID NO: 92.

22. The antibody fusion protein of claim 3, wherein the antibody targeted to the tumor cell surface antigen DLL1 has a VH of an amino acid sequence as shown in SEQ ID NO: 93, and a VL of an amino acid sequence as shown in SEQ ID NO: 94.

23. The antibody fusion protein of claim 3, wherein the antibody targeted to the tumor cell surface antigen Notch 2/3 has a VH of an amino acid sequence as shown in SEQ ID NO: 95, and a VL of an amino acid sequence as shown in SEQ ID NO: 96.

24. The antibody fusion protein of claim 3, wherein the antibody targeting to the tumor cell surface antigen endoglin has a VH with an amino acid sequence as shown in SEQ ID NO: 97, and a VL with an amino acid sequence as shown in SEQ ID NO: 98.

25. The antibody fusion protein of claim 3, wherein the antibody targeted to the tumor cell surface antigen endoglinhas a VH of an amino acid sequence as shown in SEQ ID NO: 99, and a VL of an amino acid sequence as shown in SEQ ID NO: 100.

26. The antibody fusion protein of any one of claims 2-25, wherein the fusion protein comprises a heavy chain and a light chain having the following amino acid sequences: the heavy chain shown in SEQ ID NO: 4 and the light chain shown in SEQ ID NO: 28; the heavy chain shown in SEQ ID NO: 10 and the light chain shown in SEQ ID NO: 32; the heavy chain shown in SEQ ID NO: 52 and the light chain shown in SEQ ID NO: 60; the heavy chain shown in SEQ ID NO: 58 and the light chain shown in SEQ ID NO:54; and the heavy chain shown in SEQ ID NO:58 and the light chain shown in SEQ ID NO:56.

27. A polynucleotide encoding the PD-1 mutant polypeptide of claim 1 or the antibody fusion protein of any one of claims 2-26, preferably, the polynucleotide comprises a nucleotide sequence selected from the following group consisting of: SEQ ID NO: 1, 3, 5, 7, 9, 11, 27, 31, 51, 53, 55, 57, and 59.

28. A vector comprising the polynucleotide of claim 27.

29. A host cellcomprising the vector of claim 28.

30. A pharmaceutical composition comprising a pharmaceutically acceptable carrier or a preparation, and the PD-1 mutant polypeptide or the antibody fusion protein of any one of claims 1-26.

31. Use of the PD-1 mutant polypeptide or the antibody fusion protein of any one of claims 1-26 as a PD-1-PD-L1 interaction inhibitor.

32. Use of the PD-1 mutant polypeptide or the antibody fusion protein of any one of claims 1-26as an immunomodulator.
